# EUROPEAN PATENT SPECIFICATION

(11) **EP 4 436 949 B1**
(45) Date of publication and mention of the grant of the patent: **13.08.2025**
(21) Application number: 22818649.0
(22) Date of filing: 17.11.2022
(51) Int. Cl.: C07C 67/08, C07C 67/54, C07C 69/54

(54) **PROCESS FOR THE PRODUCTION OF C6-C12-ALKYL (METH)ACRYLIC ESTERS**
VERFAHREN ZUR HERSTELLUNG VON C6-C12-ALKYL (METH)ACRYLESTERN
PROCÉDÉ DE PRÉPARATION D'ESTERS DE C6-C12-ALKYLE D'ACIDE (MÉTH)ACRYLIQUE

(30) Priority: 25.11.2021 EP 21210408
(43) Date of publication of application: 02.10.2024
(73) Proprietor: BASF SE, 67056 Ludwigshafen am Rhein (DE)
(72) Inventor: HECHLER, CLAUS, 67063 Ludwigshafen (DE); LANG, Ortmund, 67056 Ludwigshafen (DE); HOFMANN, Horst, 67056 Ludwigshafen (DE)
(74) Representative: BASF IP Association
(86) International application number: PCT/EP2022/082221
(87) International publication number: WO 2023/094252

(56) References cited:
- WO-A1-98/52904
- WO-A2-01/85666
- DE-A1- 10 246 869

## Description

The present invention relates to a process for the production of C₆₋C₁₂-alkyl (meth)acrylic esters.

(Meth)acrylic esters are generally produced by esterification of (meth)acrylic acid with alcohols in the presence of esterification catalysts. Such processes are known for example from Kirk-Othmer, Encyclopedia of Chemical Technology, Vol. 1, pages 347-348. In the case of the production of 2-ethylhexyl acrylate, this is usually carried out in the presence of a solvent and sulfuric acid.

(Meth)acrylic acid produced from acetylene by the Reppe process or by direct oxidation of propane/propene/acrolein or isobutane/isobutene/methacrolein almost inevitably contains trace amounts of acetic acid which is formed as a by-product, and is very difficult to separate from the (meth)acrylic acid. Therefore, the crude (meth)acrylic acid ester, besides unconverted alcohol, also includes small amounts of acetate ester formed by esterification of the acetic acid with the alcohol used. As the unconverted alcohol fraction is returned to the esterification step, acetate esters are inevitably enriched in the esterification reaction. It is therefore necessary to discard part of the unconverted alcohol, with the economic disadvantage of the loss of a valuable product. The present invention seeks to avoid these disadvantages.

Alkyl esters of (meth)acrylic acid are well known and are of significance, for example, as starting monomers for the preparation of aqueous polymer dispersions which find use, for example, as adhesives, paints or textile, leather and paper auxiliaries. Particularly (meth)acrylic esters having long-chain ester groups, so-called high (meth)acrylic esters, are of great commercial interest because of high demand. For applications, especially in the food or cosmetics sector, the polymer dispersions should be largely free of volatile impurities, especially free of acetate ester, to avoid costly removal of these impurities, e.g. by laborious chemical or physical desodorization. From an industrial point of view the separation of acetate ester from (meth)acrylic esters containing the same is often important for producing pure (meth)acrylic esters. The specification requirements for pure (meth)acrylates, for example, stipulate a minimum (meth)acrylate content of 99.5 wt.% and a maximum acetate content of 1500 ppm.

There is therefore a need for effective and economically viable processes for preparing higher (meth)acrylic esters with a low acetate content.

WO 01/85666 discloses a process for refining a C₁-C₈ alkyl (meth) acrylate-containing stream comprising C₁-C₈ alkyl (meth)acrylate, C₁-C₈ alkyl acetate, heavies and C₁-C₈ alkyl alcohol. As water is usually present in the crude stream to form azeotropes, the crude stream will actually be comprised of several azeotropes containing one or more of the above-mentioned components. The crude stream is introduced into a splitter distillation column to provide an overhead fraction comprising C₁-C₈ alkyl acetate, C₁-C₈ alkyl (meth)acrylate and C₁-C₈ alcohol and a bottoms fraction comprising C₁-C₈ alkyl (meth)acrylate and heavies. The splitter distillation column overhead fraction is subjected to a further distillation in a C₁-C₈ alcohol recovery column. The C₁-C₈ alcohol recovery column is operated in the presence of water to form azeotropes such that C₁-C₈ alcohol is recovered as a bottoms stream.

DE 102 46 869 relates to a process for the preparation of (meth)acrylic esters by acid-catalyzed esterification of (meth)acrylic acid with an alcohol in the presence of a polymerization inhibitor or inhibitor mixture. The recovery/purification of the (meth)acrylic ester comprises freeing the crude (meth)acrylic acid ester from acetic ester and feed alcohol in a low boiler separation column. Acetic ester is concentrated in the mixture comprising starting alcohol, acetic ester and (meth)acrylic ester withdrawn from the low boiler separation column, and discarded. The process has a high energy demand, since it includes a plurality of distillation column.

It is one object of the present invention to provide an improved process for preparing higher (meth)acrylic esters, which avoids or at least reduces the abovementioned disadvantages. The process of the invention shall thus enable more efficient and more economically viable preparation of higher (meth)acrylic esters.

Accordingly, the present invention relates to a process for the production of C₆₋C₁₂-alkyl (meth)acrylic esters, comprising an esterification step of esterifying (meth)acrylic acid with a feed C₆₋C₁₂-alkyl alcohol to obtain a crude (meth)acrylic ester, the (meth)acrylic acid containing trace amounts of acetic acid; and purification steps of purifying the crude (meth)acrylic ester,
wherein the purification steps comprise
   - introducing crude (meth)acrylic ester into the side of a low boiler column with a rectifying section disposed above the feed point of the crude (meth)acrylic ester and a stripping section disposed below the feed point;
   - withdrawing purified (meth)acrylic ester from the low boiler column;
   - withdrawing a low boiler fraction from the top of the low boiler column, the low boiler fraction comprising alcohol and acetic ester and less than 10 wt.-% of (meth)acrylic ester;
   - directing the low boiler fraction to an acetate column, the acetate column being operated at a pressure at least 50 mbar higher than the low boiler column pressure, and separating the low boiler fraction into an alcohol fraction withdrawn at the top of the acetate column and an acetic ester fraction withdrawn at the bottom of the acetate column; and
   - recycling the alcohol fraction at least partially to the esterification step;
the process comprising no recycle from the acetate column to the low boiler column.

The invention is associated with a number of advantages:
- By the process of the invention, (meth)acrylic esters are obtained in high purity of at least 99.7%, preferably at least 99.8%. In particular, the (meth)acrylic esters contain less than 500 ppm, preferably less than 400 ppm, in particular less than 300 ppm of acetic ester.
- Even with the use of crude (meth)acrylic acid, the resulting (meth)acrylates are obtained in very high purity and substantially free of acetates.
- The process of the invention consumes less heating steam than the prior art processes.
- At least 98 % by weight of the unreacted alcohol is recovered and can be recycled at least partially to the esterification step.
- The process of the invention is susceptible to being carried out without recycling an acetate-containing stream to the low boiler column.
- The process of the invention is carried out without recycling an acetate-containing stream to any column downstream of the acrylic acid separation.

Here and throughout this application the terms "(meth)acrylic acid", "(meth)acrylic ester" or "(meth)acrylate" relate to acrylic acid or the corresponding acrylic esters or acrylates and also to methacrylic acid or the corresponding methacrylic esters or methacrylates.

Here and throughout the application the term "crude (meth)acrylic acid" is understood as meaning (meth)acrylic acid whose purity is at least 90% and which contains trace amounts of acetic acid, its content being at least 0.05% by weight and at most 3 % by weight.

The crude (meth)acrylic ester is essentially anhydrous, meaning that it has a water content of less than 1 % by weight, preferably less than 10 ppm, preferably less than 5 ppm, most preferably of 1 ppm or less than 1 ppm such as less than 0.5 ppm, 0.25 or 0.1 ppm. No azeotropes are formed during distillation in the low boiler column and acetate column. To obtain a crude (meth)acrylic ester that is essentially anhydrous, water of reaction is substanially removed during esterification, and reintroduction of water during subsequent steps, e.g., a catalyst removal step or a (meth)acrylic acid removal step, is avoided.

Here and throughout this application the term "pure (meth)acrylic acid" or "prepurified (meth)acrylic acid" is understood as meaning (meth)acrylic acid whose purity is at least 99.5% by weight and which usually contains 0.01-0.2% by weight of acetic acid.

Here and throughout this application, the term "pure (meth)acrylic ester" is understood as meaning a (meth)acrylic ester whose purity is at least 99.7%, the content of acetate ester impurities being less than 500 ppm.

Here and throughout this application a "low boiler (relative to the (meth)acrylate in question)" or low-end impurity" is a substance whose boiling point is lower than the boiling point of the (meth)acrylate in question. The terms "low-end impurity" and light ends-impurity" are used synonymously.

Here and throughout this application a "high boiler (relative to the (meth)acrylate in question)" or heavy-end impurity is a substance whose boiling point is higher than the boiling point of the (meth)acrylate in question. The terms "heavy-end impurity" and high- ends impurity" are used synonymously.

Here and throughout this application, the term "distillate" defines the product withdrawn in a distillation column as side product or as top product.

The terms "acetate ester" and "acetic ester" are used synonymously.

Here and throughout the specification, the terms "wt.-%" and "% by weight" are used synonymously.

The process of the invention may be carried out in a continuous form or batchwise form, preferably in a continuous form.

The process of the invention is used for the preparation of either methacrylates or acrylates, preferably for the preparation of acrylates.

According to the invention, the process comprises at least an esterification step and a purification step, but typically may comprise further steps such as a catalyst removal step and a (meth)acrylic acid separation step.

### Esterification

The (meth)acrylic acid can be synthesized via known processes. For example, the (meth)acrylic acid used in the present invention may be one which is obtained by the direct oxidation of propane/propene/acrolein or isobutane/isobutene/meth-acrolein. The crude (meth)acrylic acid contains low boiling compounds (e.g. acetaldehyde, acrolein, water, acetic acid, propionic acid) and/or high boiling compounds (e.g. maleic acid, phenol, benzaldehyde, acrylic acid dimer) as impurities. The content of these impurities is not constant since it varies with the preparation conditions. But, the present invention is not affected by the variation of the content of the impurities.

The crude (meth)acrylic acid may comprise the following components:

| | |
|---|---|
| (Meth)acrylic acid | 90-99.9% by weight |
| Acetic acid | 0.05-3% by weight |
| Propionic acid | 0.01-1% by weight |
| Diacrylic acid | 0.01-5% by weight |
| Water | 0.05-10% by weight |
| Furfural | 0.01-0.1% by weight |
| Benzaldehyde | 0.01-0.05% by weight |
| Other aldehydes and carbonyl-containing compounds | 0.01-0.3% by weight |
| Inhibitors | 0.01-0.1% by weight |
| Maleic acid (anhydride) | 0.001-0.5% by weight |

Crude (meth)acrylic acid can be purified by multistage crystallization or, if required, by chemical treatment with an aldehyde scavenger and distillation.

A pure (meth)acrylic acid may include the following components.

| | |
|---|---|
| (Meth)acrylic acid | 99.5-99.9% by weight |
| Acetic acid | 0.01-0.15% by weight |
| Propionic acid | 0.005-0.05% by weight |
| Diacrylic acid | 0.01-0.2% by weight |
| Water | 0.01-0.1% by weight |

The crude as well as the pure or prepurified (meth)acrylic acid are typcially stabilized with a polymerization inhibitor or polymerization inhibitor mixture against the premature polymerization of the (meth)acrylic acid. With regard to polymerization inhibitors (stabilizers) suitable and preferred for this purpose, reference is made to the general observations made below in relation to suitable and preferred stabilizers.

Both crude (meth)acrylic acid as well as pure (meth)acrylic acid can be used for the process according to the invention. Preference is given to use crude (meth)acrylic acid.

For the process according to the invention, alcohols containing 6 to 12, preferably 8 to 12 carbon atoms are preferably used in the esterification step, for example, C₆-C₁₂-alkanols such as n-hexanol, n-heptanol, n-octanol, n-decanol, n-dodecanol (lauryl alcohol), 2-ethylhexanol, 2-propylheptanol, C₆-C₁₂-cycloalkanols such as cyclohexanol, cyclooctanol, cyclododecanol, but also glycol ether such as ethylene glycol monomethyl ether, ethylene glycol monoethyl ether, diethylene glycol monomethyl ether or diethylene glycol monoethyl ether, preferably n-octanol and 2-ethylhexanol, 2 ethylhexanol being particularly preferred.

The 2-ethylhexanol used in the process of the invention can contain the following components, for example:

| | |
|---|---|
| 2-ethylhexanol | at least 95%by weight |
| n-butyraldehyde | up to 0.1 % by weight |
| 4-heptanone | up to 0.2% by weight |
| 3-heptanone | up to 0.1 % by weight |
| 4-heptanol | up to 0.2% by weight |
| 2-ethylhexanal | up to 0.2% by weight |
| 3-methylheptan-4-ol | up to 0.5% by weight |
| 2-ethyl-methyl-pentanol | up to 1% by weight |
| 2-ethylhex-3-enol | up to 0.1 % by weight |
| 2-ethylhexenal | up to 0.5% by weight |
| n-butyl-2-ethylhexylethylether | up to 0.1 % by weight |
| other ethers | up to 0.5% by weight |
| water | up to 1.0% by weight |

The molar ratio of the (meth)acrylic acid to alcohol in the reaction mixture is typically in the range from 1:0.7-2.0, preferably 1:0.7-1.7, particularly preferably 1:0.7-1.3. Typically, the (meth)acrylic acid is used in deficiency, based on the amount of the alcohol used.

Preferably, the esterification is carried out in the presence of at least one catalyst.

Preference is given to an acid catalyst, especially a strongly acidic catalyst. Suitable catalysts include sulfuric acid, alkyl or aryl sulfonic acid, for example methanesulfonic acid, trifluoromethanesulfonic acid, benzenesulfonic acid, para-toluenesulfonic acid, dodecylbenzenesulfonic acid or a strongly acidic ion exchanger. The catalyst content usually is 0.1-10, preferably 0.3-5% by weight, based on the reaction mixture present therein.

The esterification is usually carried out at 70-150°C, preferably 80 to 130° C and at a pressure of 100 mbar to normal pressure, preferably 200 to 800 mbar, particularly preferably 250 to 700 mbar. In the case of several reaction regions, the reaction temperature is preferably set in such a way that it rises along the cascade.

The esterification takes place in conventional reactors or columns. Usually the esterification takes place in a reaction zone consisting of one or more reaction regions, for example a reactor cascade of two to four, preferably two to three reactors. In the embodiment of the invention having a plurality of reaction regions, it is advantageous to cascade these. If more than one reaction region is created within one and the same reactor (e.g. by the use of separating sheets of metal), the number of reaction regions can also be greater than 4.

The esterification is carried out in the presence of at least one polymerization inhibitor or inhibitor mixture.

Suitable polymerisation inhibitors include alkylphenols, for example o-, m- or p-cresol (methylphenol), 2-tert-butyl-4-methylphenol, 6-tert-butyl-2,4-dimethylphenol, 2,6-di-tert-butyl-4-methylphenol, 2-tert-butylphenol, 4-tert-butylphenol, 2,4-di-tert-butylphenol, 2-methyl-4-tert-butylphenol, 4-tert.-butyl-2,6-dimethylphenol, or 2,2'-methylene-bis-(6-tert-butyl-4-methylphenol), hydroxyphenols, for example hydroquinone, 2-methylhydroquinone, 2,5-di-tert-butylhydroquinone, catechol (1,2-dihydroxybenzene) or benzoquinone, aminophenols, such as para-aminophenol, nitrosophenols, such as para-nitrosophenol, alkoxyphenols, for example 2-methoxyphenol (guaiacol, pyrocatechol monomethyl ether), 2-ethoxyphenol, 2-isopropoxyphenol, 4-methoxyphenol (hydroquinone monomethyl ether), mono- or di-tert-butyl-4-methoxyphenol, tocopherols, such as e.g. alpha-tocopherol and 2,3-dihydro-2,2-dimethyl-7-hydroxybenzofuran (2,2-dimethyl-7-hydroxycoumaran), N-oxyls such as 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethylpiperidine-N-oxyl, 2,2, 6,6-tetramethyl-piperidine-N-oxyl, 4,4',4"-tris (2,2,6,6-tetramethyl-piperidine-N-oxyl) phosphite or 3-oxo-2,2, 5,5-tetramethyl-pyrrolidine-N-oxyl, aromatic amines or phenylenediamines, such as N, N-diphenylamine, N-nitrosodiphenylamine, N,N'-dialkyl-para-phenylenediamine, where the alkyl radicals may be the same or different and each independently consist of 1 to 4 carbon atoms and may be straight-chain or branched, hydroxylamines, such as N, N-diethylhydroxylamine, phosphorus-containing compounds, such as triphenylphosphine, triphenylphosphite, hypophosphorous acid or triethylphosphite, sulfur-containing compounds, such as diphenyl sulfide or phenothiazine, optionally in combination with metal salts, such as the chlorides, dithiocarbamates, sulfates, salicylates or acetates of copper, manganese, cerium, nickel or chromium. Mixtures of stabilizers can of course also be used. Typically one or more, for example two or three, of the aforementioned polymerization inhibitors are used. Preference is given to using one or two of the aforementioned polymerization inhibitors.

Phenothiazine or another stabilizer which shows the same effectiveness is preferably used.

To further support the stabilization, an oxygen-containing gas, preferably air or a mixture of air and nitrogen (air having a low oxygen content), may be present. This oxygen-containing gas is preferably metered into the reaction mixture and / or into the bottom region of a column and / or into a circulation evaporator.

The reaction regions are equipped with distillation units, advantageously with a common distillation unit. In this case, the reflux from the distillation unit is passed into the first reaction region.

The distillation unit is of a design known per se and has the conventional internals. Suitable column internals are in principle all conventional internals, for example trays, stacked packings and/or dumped packings. Among the trays, bubble trays, sieve trays, valve trays, Thormann trays and/or dual-flow trays are preferred; among the dumped packings, those comprising rings, coils, saddles, Raschig, Intos or Pall rings, barrel or Intalox saddles, Top-Pak etc. or braids are preferred.

In general, from 5 to 20 theoretical plates are sufficient to achieve a desirable degree of separation.

The heat can be supplied via internal and/or external heat exchangers of conventional design and/or via jacket heating (the heat transfer medium used is advantageously steam). The heat is preferably supplied via external circulation evaporators with natural or forced circulation. The thorough mixing of the reaction mixture is effected in a known manner, for example by stirring, pumped circulation or natural circulation. The water formed during esterification is discharged via the column together with the low-boiling component or with solvents which form an azeotrope with water and have a boiling point at normal pressure of up to 130°C and condensed in a condenser of known design. Preferably no extraneous solvent is used.

In a preferred embodiment, the esterification is carried out in the presence of an intrinsic azeotropic entrainer. The azeotropic entrainer preferably is an olefinic compound, generally an olefin having the same carbon number as the feed alcohol. These olefins are intrinsically generated at low rate in a dehydration side reaction and possibly subsequent isomerisation of the alcohol during the course of the esterification. In the case of 2-ethylhexyl alcohol the olefinic by-products are octenes, e.g. 2-ethylhex-1-ene, 3-methylhept-2-ene, 3-methylhept-3-ene. The azeotropic entrainer forms a low boiling azeotrope with water that distills up to the top of the column above the esterification stage, entraining the water.

The condensate decomposes into a water phase, which is discharged, and an organic phase, mainly consisting of olefinic entrainer, 2-ethylhexanol, 2-ethylhexyl acetate and propionate. The by-products usually are partly, preferably 5-10%, removed and partly re-cycled as reflux to the uppermost bottom of the column in combination with part of the fresh alcohol.

In a preferred embodiment part of the organic phase which has not been discharged is wholly or partly returned directly to the esterification, bypassing the column, and the fresh alcohol alone or, if appropriate, together with part of this organic phase is used as reflux.

In another preferred embodiment at least part of the organic phase is metered into the circulation evaporators, if these are natural circulation evaporators, thereby supporting the circulation.

For stabilization, a solution of one or more stabilizers is added. With regard to polymerization inhibitors (stabilizers) suitable and preferred for this purpose, reference is made to the general observations made above in relation to suitable and preferred stabilizers. Preference is given to phenothiazine, methylene blue, hydroquinone, hydroquinone monomethyl ether, alkyl-substituted phenols or N-oxyl compounds or mixtures thereof. Typically, a solution of one or more stabilizers in 2-ethylhexanol is sprayed into the condenser and/or added to the reflux, the total amount of the stabilizer(s) being 0.01-10%, preferably 0.2-5% and particularly preferably 0.3-1%. In particular, preference is given to a solution of 0.1-1 % by weight, preferably 0.2-0.8 and particularly preferably 0.3-0.7% by weight of phenothiazine and 0.001-0.1 % by weight, preferably 0.002-0.05% by weight of 4-hydroxy-2,2,6,6-tetramethylpiperidine-N-oxyl in 2-ethylhexanol.

To further support the circulation, an inert gas, preferably an oxygen-containing gas, particularly preferably air or a mixture of air and nitrogen (air having a low oxygen content, e.g. an oxygen content of 5 to 10 vol.-%) can be introduced into the circulation, preferably into the circulation evaporator, for example in amounts of 0.1-1, preferably 0.2-0.8 and particularly preferably 0.3-0.7 m³/ m³h, based on the volume of the reaction mixture. Of course, the inert gas can also be passed as a cover over or into the reaction mixture.

### Catalyst removal step

A stream coming from the esterification and mainly comprising (meth)acrylic ester, unconverted alcohol, (meth)acrylic acid, light-ends, acetate ester, inhibitors, catalyst and heavy boilers (oxyesters), is fed into a catalyst separation column, where the stream is separated into a bottom fraction and an overhead fraction.

The overhead product (condensate), which consists mainly of (meth)acrylic ester and low boilers can be partly, preferably 3-10%, returned to the column as reflux, and partly, preferably 15-30%, optionally after addition of stabilizer (see above), introduced into a condenser and the remainder fed to the acrylic acid separation.

In a preferred embodiment, the condensate and, if necessary, the stabilizer solution is sprayed onto the upper tube sheet and the metal surface of the condenser hood via a nozzle located in the head of the condenser, preferably in co-current flow with the vapour.

The bottom fraction essentially comprises catalyst, inhibitors, (meth)acrylic ester and heavy boilers. For the recuperation of the valuables contained in the bottom fraction, it can be completely or partially returned to the reaction zone, preferably to the first reactor if a cascade is used, and/or fed to a residue distillation and/or residue cleavage which may be present. Preferably 1 to 30% are fed to the residue cleavage.

The distillation column is of a design known per se and has the customary internals and is equipped with a circulation evaporator and condenser. The feed is preferably fed in the bottom area, the bottom temperature here is 130-160°C and the head pressure 50-200 mbar.

The column preferably has only a few trays or even no internals active for thermal separation. It is preferably equipped with one or more droplet precipitators (spray precipitator) to minimize droplet entrainment of heavy boiling components, namely catalyst, by the vapor stream.

It may be advantageous to recycle the condensate to the column, a part being fed into the column head, advantageously the column head is sprayed from below, a part is sprayed onto the droplet precipitator from above and/or a part is sprayed to the droplet precipitator from below.

In a preferred embodiment, a falling-film, thin-film or wiped-film evaporator, for example a Luwa, Rotafilm or Sambay evaporator, which is equipped, for example, with a demister as spray protection, can be used.

In a further preferred embodiment, the process of the invention also comprises a (meth)acrylic acid removal step prior to introducing the crude (meth)acrylic ester into the low boiler column.

### (Meth)acrylic acid separation

The overhead stream of the catalyst separation is fed into a distillation unit consisting of a column of conventional construction, a circulation evaporator and a condenser of conventional construction and separated into a bottom stream which is largely free of (meth)acrylic acid ((meth)acrylic acid content below 0.1 % by weight) and a (meth)acrylic acid-containing head stream. The bottom stream mainly consists, preferably more than 90%, of (meth)acrylic ester, the overhead product essentially comprises unreacted alcohol, (meth)acrylic ester, (meth)acrylic acid and light-ends. The overhead product is referred to as crude (meth)acrylic ester . The temperature at the bottom is usually from 130 to 160°C and the pressure at the top is usually from 50 to 130 mbar.

The column may contain separation-active internals mentioned in the esterification step. Among the separation-active internals mentioned there, especially preference is given to the trays. A column with 20-40 theoretical trays is preferred.

The feed is preferably in the upper half.

The overhead stream (condensate) can be partially, preferably 10-20%, fed back to the column as reflux, partially, preferably 5-10%, introduced at the top of the column, preferably injected, partially, preferably 7-14%, sprayed into the condenser head and the rest can be returned to the reaction zone, preferably to the first reactor if a cascade is used.

In an advantageous embodiment, part of the reflux is used to spray the column head and the remaining part of the reflux is applied to the separating internals via a distributor device or a nozzle.

A stabilizer is fed into the condenser, preferably into the condenser head, e.g. by injection. Suitable stabilizers are those mentioned in the esterification step above. Preference is given to a solution of 0.1-1% by weight, preferably 0.2-0.8% by weight and particularly preferably 0.3-0.7% by weight of phenothiazine and 0.001-0.1, preferably 0.002-0.05% by weight of 4-hydroxy-2,2,6,6-tetramethylpiperidine N-oxyl in 2-ethylhexyl acrylate or 2-ethylhexanol.

The stabilizer can also be fed into the condensator together with the recycled condensate. In a preferred embodiment, the condensate and optionally the stabilizer solution is sprayed onto the condenser surface via a nozzle located in the head of the condenser, preferably in co-current flow with the vapour.

### Purification

The crude (meth)acrylic ester stream is fed into the side of a low boiler column with a rectifying section disposed above the feed point of the crude (meth)acrylic ester and a stripping section disposed below the feed point. The stream contains the target ester as main component as well as unreacted alcohol, acetate ester and light-ends. When the starting alcohol is 2-ethylhexanol, impurities are 2-ethylhexanol, 2-ethylhexyl acetate, 2-ethylhexyl propionate and light-ends.

Purified (meth)acrylic ester is taken off from the low boiler column. Generally, the purified (meth)acrylic ester has a purity is at least 99%, the content of acetate ester impurities being less than 500 ppm.

In the preferred embodiment, the purified (meth)acrylic ester is withdrawn from the bottoms of the low boiler column, and the purified (meth)acrylic ester is introduced into a finishing column. Overhead from the finishing column, pure (meth)acrylic ester is withdrawn which is condensed. The bottoms from the finishing column is essentially (meth)acrylic ester and high-boiler, which is recycled at least partly to to the catalyst removal column to increase the yield.

In an other, still preferred embodiment, the purified (meth)acrylic ester is withdrawn as a side stream from the low boiler column, preferably from the stripping section thereof, and the bottoms from the low boiler column which is essentially (meth)acrylic ester and high-boiler is recycled at least partly to to the catalyst removal column. In other words, the low boiler separation column and finishing column are configured as a single distillation column. Preferably, the purified (meth)acrylic ester withdrawn as a side stream from the low boiler column meets the specification requirements for pure (meth)acrylic ester, and is not subjected to further distillation or refinement.

A low boiler fraction is withdrawn from the top of the low boiler column. The low boiler fraction is fed into an acetate column. According to the invention, the low boiler fraction comprises less than 10 wt.-%, preferably less than 5 wt.%, of (meth)acrylic ester. This means, the separation efficiency of the rectifying section of the low boiler column must be sufficient to adequately limit the quantity of (meth)acrylic ester carried over to the low boiler fraction. Separation efficiency is largely determined by the height of the rectifying section and the column internals. The process comprises no recycle from the acetate column to the low boiler column.

Preferably, the rectifying section of the low boiler column comprises at least 8 theoretical plates, e.g. in the range from 10 to 20 Preferably, the number of theoretical plates in the rectifying section of the low boiler column is greater as in the stripping section, e.g. greater by a factor of from 1.2 to 4 More preferably, the number of theoretical plates in the rectifiying section of the low boiler column is greater by a factor of from 1.5 to 3.

Especially, the low boiler column has a number of theoretical plates in the range from 13 to 30 the side feed point for the crude (meth)acrylate containing stream is arranged at a theoretical plate in the region commencing at least 4theoretical plates above the bottom most theoretical plate and ending at least 8 theoretical plates below the uppermost theoretical plate. The side offtake point for the pure (meth)acrylate is arranged at a theoretical plate in the region commencing at least 1 theoretical plate above the bottommost theoretical plate and ending at least 12 theoretical plates below the uppermost theoretical plate.

The column internals used may in principle be all standard internals, for example trays, structured packing elements and/or random packing elements.

Here and throughout the application the term "packing" means solid or hollow bodies of predetermined size, shape, and configuration used as column internals to provide surface area for the liquid to allow mass transfer at the liquid-vapor interface during countercurrent flow of two phases. Two broad classes of packings are "random" and "structured".

"Random packing" means packing wherein individual members do not have any particular orientation relative to each other or to the column axis. Random packings are small, hollow structures with large surface area per unit volume that are loaded at random into a column.

"Structured packing" means packing wherein individual members have specific orientation relative to each other and to the column axis. Structured packings usually are made of thin metal foil, expanded metal or woven wire screen stacked in layers or as spiral windings. The term "surface area density" means the surface area of the structured packing per unit volume of the structured packing, and usually is expressed in terms of m²/m³ of the volume occupied by the packing.

Among the trays, preference is given to bubble-cap trays, sieve trays, valve trays, Thormann trays and/or dual-flow trays; among the random packing elements, preference is given to those comprising rings, helices, saddles, Raschig, Intos or Pall rings, Berl or Intalox saddles, or braids.

More preferably, the rectifying section of the low boiler column comprises one or more structured packing elements. Structured packing elements are made up of a multiplicity of individual layers of packing elements, such as metal sheets, expanded metals and wire fabrics, which are disposed vertically to one another in a regular structure and are usually held together in a composite by attachments such as metal wires, thin metal rods or metal sheet strips. Usually the structured packing elements themselves have a geometric structuring, for example in the form of folds or circular holes of from about 4 to 6 mm in diameter. The openings act to increase the flood limit of the packing and to make a higher column load possible. Examples are packings of the types "Mellapak", CY and BX from Sulzer AG, CH-8404 Winterthur, or types A3, BSH or B1 from Montz GmbH, D-40723 Hilden. The folds of the packing elements of these packings run linearly and at an angle of from about 30° to 45° to the longitudinal axis of the packing. The foldings of the packing elements lead to a cross-channel structure within the structured packing. Structured packings are usually provided as individual packing layers which are then arranged in the column stacked one above the other. The packing layers usually have a height of from about 0.17 m to about 0.30 m.

The structured packing layer may have an internal geometry which varies over its height. A structured packing is preferred in which the structured packing has one or more structured packing elements having a surface area density of at least 100 m²/m³, preferably at least 200 m²/m³, more preferably at least 300 m²/m³. In one embodiment, the structured packing has a surface area density of about 250 m²/m³ to 350 m²/m³, and includes a plurality of corrugated plates disposed in parallel relation.

The structured packing elements preferably having a total height of at least 6 m, preferably at least 8 m.

Commercially available structured packing elements are e.g. Sulzer Mellapak 250Y; Raschig Super-Pak 350Y, Koch-Glitsch Flexipac HC 350Y.

The heat can be supplied via internal and/or external heat exchangers of conventional design and/or via jacket heating (the heat transfer medium used is advantageously steam). The heat is preferably supplied via external circulation evaporators with natural or forced circulation. The thorough mixing of the reaction mixture is effected in a known manner, for example by stirring, pumped circulation or natural circulation.

In general, the pressure is from 40 to 200 mbar, preferably from 50 to 100 mbar, measured at the top of the low boiler column. The temperature is from 120 to 180 °C, preferably from 130 to 160 °C, measured at the bottom of the low boiler column.

A stabilizer solution is typically added to the purified (meth)acrylic ester. With regard to polymerization inhibitors (stabilizers) suitable and preferred for this purpose, reference is made to the general observations made above in relation to suitable and preferred stabilizers. Preferably a solution of phenothiazin in 2-ethylhexanol or 2-ethylhexylacrylat is preferably added to the side take-off.

### Finishing column

Optionally, the process according to the invention invoves a finishing column. In an embodiment mentioned above, the purified (meth)acrylic ester is withdrawn from the bottoms of the low boiler column, and the purified (meth)acrylic ester is introduced into a finishing column.

The finishing column is of a design known per se and has the customary internals.

Useful column internals include in principle all common internals, for example trays, structured packings and/or random packings. In general, 2 to 6 theoretical plates are sufficient.

The pressure is usually from 40 to 200 mbar measured at the top of the finishing column B, the distillation temperature is from 120 to 180 °C, measured at the bottom of the finishing column B.

### Acetate column

The low boilers from the low boiler are fed, preferably continuously, to an acetate column in which a fraction comprising essentially starting alcohol is separated from a stream comprising substantially acetate ester.

According to the invention, the acetate column being operated at a pressure at least 50 mbar higher than the low boiler column pressure. The operating pressure in the acetate column is at least 50 mbar, preferably at least 100 mbar, more preferably at least 200 mbar higher than the finishing column pressure, measured in each case at the top of the finishing colum and acetate column. Viewed alternatively, the acetate column is preferably being operated at a pressure 2 to 10 times higher than the low boiler column pressure. While alcohol and acetic ester have similar boiling points at the low boiler column pressure, the relative boiling points change to a significant degree, as the pressure to which the low boiler fraction is exposed is changed. Acetic ester concentrates in the distillate during distillation at the low boiler column pressure, and upon distillation of the distillate at a higher pressure, will tend to concentrate in the bottoms.

For safe operation of the acetate column at a higher pressure it is essential that the low boiler fraction comprises less than 10 wt.-% of (meth)acrylic ester, preferably less than 5 wt.-% of (meth)acrylic ester.

The stream comprising essentially starting alcohol is at least partly recycled to the esterification step. The stream comprising essentially acetate ester is discharged.

The acetate column is a rectifying column of known design, for example tray columns or columns with random packing or columns with structured packing. With regard to separation-active internals suitable and preferred for this purpose, reference is made to the general observations made above in relation to suitable and preferred internals suitable for the finishing column. In particular, columns with structured packing are preferred.

Preference is given to an operating mode where the temperature in the acetate column is 120 to 190 °C, preferably about 160 °C, measured at the bottom of the acetate column. The pressure measured at the top of the column is in the range from 90 to 1000mbar, preferably in the range between 200 and 400 mbar.

The invention is illustrated in detail by the examples described hereinafter. These examples should not be understood in such a way that they restrict the invention.

In the examples which follow, the following abbreviations are used:

| | |
|---|---|
| AA | acrylic acid |
| 2EHA | 2-ethylhexyl acrylate |
| EHOL or 2EHOL | 2-ethylhexanol |
| 2EHOAc | 2-ethylhexyl acetate |
| PTSA | para-toluene sulfonic acid |
| l.b. | low ends |
| h.b. | heavy ends |
| % b.w. | % by weights |

Unless otherwise stated, the parts, percentages and ppm data given herein relate to parts by weight,% by weight and ppm by weight.

The operating modes are illustrated using data from a thermodynamic simulation of a complete plant for the production of 2-ethylhexyl acrylate.

The thermodynamic simulation of the process was carried out using proprietary software. Commercial software is available, such as Aspen Plus^{®} program from Aspen Technology, Inc., of Cambridge, Massachusetts, that is expected to yield comparable results. Aspen Plus^{®} is a comprehensive simulation software which is used for the modeling, simulation and optimization of chemical processes and plants in industry. Aspen has comprehensive modeling data banks for modeling the basic operations and also materials data banks for the materials properties of many different substances. The properties of mixtures are calculated by Aspen Plus^{®} by means of various thermodynamic models from the materials data of the pure substances

The simulation was carried out for the process of the invention and a reference process. In all simulations, the stream discharged from the acrylic acid separation had the same composition.

### Figures

Figure 1 is a diagram of a process according to example 1.
Figure 2 is a diagram of a process according to the invention according to example 2, comprising a low boiler column A and finishing column B that are physically separated, and acetate column D.
Figure 3 is a diagram of a reference process comprising four columns, comprising a low boiler column A, finishing column B, low boiler distillation column C and acetate column D, each column being physically separated.

### Example 1:

### Purification of 2-EHA using two columns (finishing A and acetate column D)

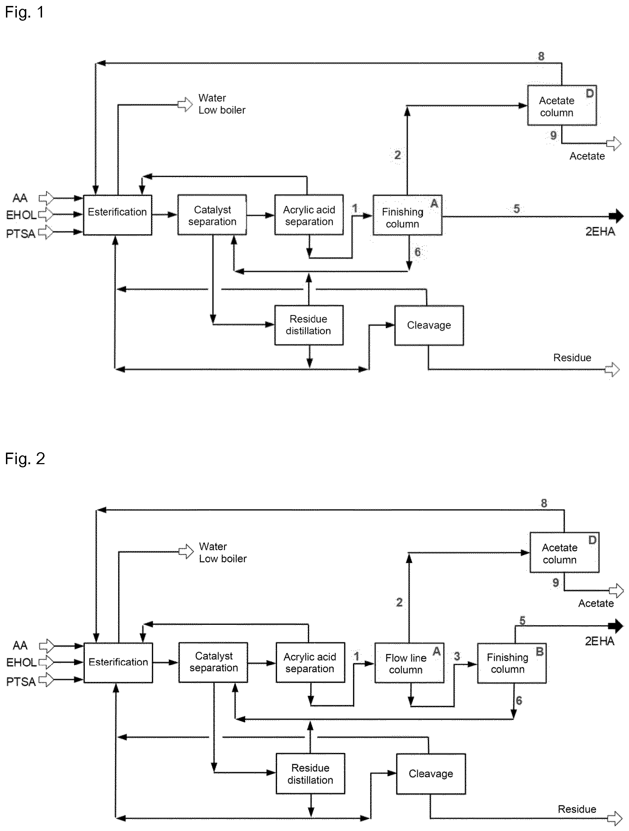

As shown in Fig. 1, a crude 2EHA stream **1** comprising as impurities 2EHOL, 2-EHOAc, heavy ends and low ends was fed into a finishing column **A.** The finishing column **A** included 24 theoretical plates; the feed plate was the 8^{th} theoretical plate from the bottom, the overhead pressure was 60 mbar and the bottom temperature was 147 °C. A liquid stream **5** consisting essentially of 2EHA was taken from the stripping section of the finishing column **A.** A stream **6** consisting essentially of 2EHA and heavy ends impurities was withdrawn from the stripping section of column **A** and sent to a catalyst separation column. A low boiler stream **2** comprising 2EHA, 2EHOL and 2EHOAc was withdrawn from the top of the finishing column **A** and fed to an acetate column **D.** The acetate column **D** included 24 theoretical plates, the feed plate was the 15^{th} theoretical plate from the bottom, the bottom temperature was 162 °C and the overhead pressure was 314 mbar. A stream **8** consisting mainly of 2EHOL and 2EHOAc was taken from the top of the acetate column **D** and returned to an esterification reactor and a bottom product **9** consisting mainly of 2EHOAc and 2EHA was removed from the acetate column **D.** The composition of the streams **1, 2, 5, 6, 8** and **9** are shown in table 1 below.

**Table 1:**

| | | **finishing column A** | | | | **acetate column D** | | |
|---|---|---|---|---|---|---|---|---|
| | | **1** | **2** | **6** | **5** | **2** | **8** | **9** |
| 2EHOL | % b.w. | 1.10 | 75.67 | 0.01 | 0.05 | 75.67 | 88.37 | 0.28 |
| 2EHOAc | % b.w. | 0.36 | 20.33 | 0.02 | 0.08 | 20.33 | 11.62 | 71.98 |
| 2EHA | % b.w. | 98.16 | 4.00 | 90.87 | 99.84 | 4.00 | 0.00 | 27.74 |
| h.e. | % b.w. | 0.37 | 0.00 | 8.97 | 0.01 | 0.00 | 0.00 | 0.00 |
| l.e. | % b.w. | 0.01 | 0.01 | 0.14 | 0.00 | 0.01 | 0.01 | 0.00 |
| mass flow rate | kg/h | 5000 | 69 | 200 | 4731 | 69 | 59 | 10 |

| | | | | | | | | |
|---|---|---|---|---|---|---|---|---|
| Amount of heating steam: 1347 kg/h. | | | | | | | | |

### Example 2:

### Purification of 2EHA using three columns (low boiler separation column A, finishing column B, and acetate column D)

As shown in Fig. 2, a crude 2EHA stream **1** comprising as impurities 2EHOL, 2EHOAc, heavy ends and low ends was fed into a low boiler separation column **A.** The low boiler separation column **A** included 20 theoretical plates; the feed plate was the 5^{th} theoretical plate from the bottom, the overhead pressure was 40 mbar and the bottom temperature was 126 °C. A liquid stream **3** comprising mainly 2EHA and heavy ends impurities was taken from the stripping section of the low boiler separation column **A** and fed to a finishing column **B.** The finishing column **B** included 6 theoretical plates; the feed plate was the 1^{st} theoretical plate from the bottom, the bottom temperature was 129 °C and the overhead pressure was 22 mbar. A stream **6** comprising mainly 2EHA and heavy ends impurities was withdrawn from the stripping section of the finishing column **B** and sent to a catalyst separation column. A stream **5** comprising mainly the target ester 2EHA was taken from the top of the finishing column **B.** A low boiler stream **2** comprising mainly 2EHOL, 2EHOAc and 2EHA was withdrawn from the top of the low boiler separation column **A** and fed to an acetate column **D.** The acetate column **D** included 24 theoretical plates, the feed plate was the 15^{th} theoretical plate from the bottom, the bottom temperature was 162 °C and the overhead pressure was 314 mbar. A stream **8** comprising mainly 2EHOL and 2EHOAc was taken from the top of the acetate column **D** and returned to an esterification reactor and a bottom product **9** consisting essentially of 2EHOAc and 2EHA was removed from the acetate column **D.** The composition of the streams **1, 2, 3, 5, 6, 8** and **9** are shown in table 2 below.

**Table 2**

| | | **low boiler column A** | | | **finishing column B** | | |
|---|---|---|---|---|---|---|---|
| | | **1** | **2** | **3** | **3** | **6** | **5** |
| 2EHOL | % b.w. | 1.10 | 75.65 | 0.08 | 0.08 | 0.03 | 0.08 |
| 2EHOAc | % b.w. | 0.36 | 20.34 | 0.09 | 0.09 | 0.04 | 0.09 |
| 2EHA | % b.w. | 98.16 | 4.00 | 99.45 | 99.45 | 90.52 | 99.83 |
| h.e. | % b.w. | 0.37 | 0.00 | 0.38 | 0.38 | 9.25 | 0.00 |
| l.e. | % b.w. | 0.01 | 0.01 | 0.01 | 0.01 | 0.17 | 0.00 |
| mass flow rate | kg/h | 5000 | 67 | 4933 | 4933 | 200 | 4733 |

**Table 2 (continuation)**

| | | **acetate column D** | | |
|---|---|---|---|---|
| | | **2** | **8** | **9** |
| 2EHOL | % b.w. | 75.65 | 88.78 | 0.31 |
| 2EHOAc | % b.w. | 20.34 | 11.21 | 72.73 |
| 2EHA | % b.w. | 4.00 | 0.00 | 26.96 |
| h.e. | % b.w. | 0.00 | 0.00 | 0.00 |
| l.e. | % b.w. | 0.01 | 0.01 | 0.00 |
| mass flow rate | kg/h | 67 | 57 | 10 |

| | | | | |
|---|---|---|---|---|
| Amount of heating steam: 1526 kg/h. | | | | |

### Example 3 (Reference):

### Purification of 2EHA using four columns (low boiler separation column A, finishing column B, low boiler distillation C and acetate column D)

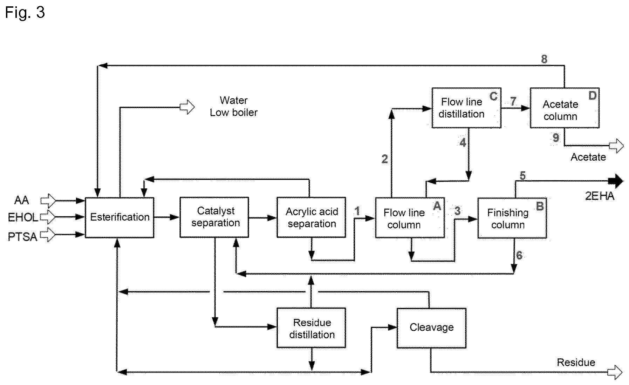

As shown in Fig. 3, a stream **1** comprising 2EHA, 2EHOL, 2EHOAc, heavy ends impurities and low ends impurities was fed into a low boiler separation column **A.** At least one part of a bottom stream **4** of crude 2EHA from a low boiler distillation **C** was also fed into the low boiler distillation **A.** The low boiler separation column **A** included 12 theoretical plates; the feed plate was the 5^{th} theoretical plate from the bottom, the overhead pressure was 40 mbar and the bottom temperature was 123 °C. A liquid stream **3** comprising mainly 2EHA and heavy ends impurities was taken from the stripping section of the low boiler separation column **A** and fed to a finishing column **B.** The finishing column **B** included 6 theoretical plates; the feed plate was the 1^{st} theoretical plate from the bottom, the bottom temperature was 129 °C and the overhead pressure was 22 mbar. A stream **6** comprising mainly 2EHA and heavy ends impurities was withdrawn from the stripping section of the finishing column **B** and sent to a catalyst separation column. A stream **5** consisting essentially of the target ester 2EHA was taken from the top of the finishing column **B.** A low boiler stream **2** comprising mainly 2EHA, 2EHOL and 2EHOAc was withdrawn from the top of the low boiler separation column **A** and fed to the low boiler distillation **C.** The low boiler distillation **C** included 18 theoretical plates, the feed plate was the 1^{st} theoretical plate from the bottom, the bottom temperature was 120 °C and the overhead pressure was 40 mbar. An overhead stream **7** of the low boiler distillation **C** consisting mainly of 2EHOL and 2EHOAc was fed to an acetate column **D.** The acetate column **D** included 24 theoretical plates, the feed plate was the 15^{th} theoretical plate from the bottom, the bottom temperature was 162 °C and the overhead pressure was 314 mbar. A stream **8** consisting essentially of 2EHOL and 2EHOAc was taken from the top of the acetate column **D** and returned to an esterification reactor and a bottom product **9** consisting essentially of 2EHOAc and 2EHA was removed from the acetate column **D.** The main constituents of the streams **1** to **9** expressed in % by weight and the hourly mass flow rate (kg/h), are given in Table 3.

**Table 3:**

| | | **low boiler column A** | | | | **finishing column B** | | |
|---|---|---|---|---|---|---|---|---|
| | | **1** | **2** | **3** | **4** | **3** | **6** | **5** |
| 2EHOL | % b.w. | 1.10 | 39.71 | 0.09 | 2.55 | 0.09 | 0.03 | 0.09 |
| 2EHOAc | % b.w. | 0.36 | 10.29 | 0.10 | 1.08 | 0.10 | 0.05 | 0.11 |
| 2EHA | % b.w. | 98.16 | 50.00 | 99.42 | 96.36 | 99.42 | 90.51 | 99.80 |
| h.e. | % b.w. | 0.37 | 0.00 | 0.38 | 0.00 | 0.38 | 9.25 | 0.00 |
| I.e. | % b.w. | 0.01 | 0.00 | 0.01 | 0.00 | 0.01 | 0.17 | 0.00 |
| mass flow rate | kg/h | 5000 | 132 | 4934 | 66 | 4934 | 200 | 4734 |

**Table 3 (continuation)**

| | | **low boiler distillation C** | | | **acetate column D** | | |
|---|---|---|---|---|---|---|---|
| | | **2** | **4** | **7** | **7** | **8** | **9** |
| 2EHOL | % b.w. | 39.71 | 2.55 | 76.58 | 76.58 | 90.14 | 0.41 |
| 2EHOAc | % b.w. | 10.29 | 1.08 | 19.41 | 19.41 | 9.85 | 73.13 |
| 2EHA | % b.w. | 50.00 | 96.36 | 4.00 | 4.00 | 0.00 | 26.46 |
| h.e. | % b.w. | 0.00 | 0.00 | 0.00 | 0.00 | 0.00 | 0.00 |
| l.e. | % b.w. | 0.00 | 0.00 | 0.01 | 0.01 | 0.01 | 0.00 |
| mass flow rate | kg/h | 132 | 66 | 66 | 66 | 56 | 10 |

| | | | | | | | |
|---|---|---|---|---|---|---|---|
| Amount of heating steam: 1726 kg/h | | | | | | | |

Modeling shows that the prior art process requires more heating steam than the process of the invention. The purification method of examples 1 and 2 consumes 22% and 12% less heating steam than that of example 3.

## Claims

1. A process for the production of C₆₋C₁₂-alkyl (meth)acrylic esters, comprising an esterification step of esterifying (meth)acrylic acid with an C₆₋C₁₂-alcohol to obtain a crude (meth)acrylic ester, the (meth)acrylic acid containing trace amounts of acetic acid; and purification steps of purifying the crude (meth)acrylic ester,
wherein the purification steps comprise
- introducing crude (meth)acrylic ester into the side of a low boiler column with a rectifying section disposed above the feed point of the crude (meth)acrylic ester and a stripping section disposed below the feed point;
- withdrawing purified (meth)acrylic ester from the low boiler column;
- withdrawing a low boiler fraction from the top of the low boiler column, the low boiler fraction comprising alcohol and acetic ester and less than 10 wt.-% of (meth)acrylic ester;
- directing the low boiler fraction to an acetate column, the acetate column being operated at a pressure at least 50 mbar higher than the low boiler column pressure, and separating the low boiler fraction into an alcohol fraction withdrawn at the top of the acetate column and an acetic ester fraction withdrawn at the bottom of the acetate column; and
- recycling the alcohol fraction at least partially to the esterification step;
the process comprising no recycle from the acetate column to the low boiler column.

2. The process of claim 1, wherein said crude (meth)acrylic ester is essentially anhydrous.

3. The process of claim 1 or 2, the acetate column being operated at a pressure 2 to 10 times higher than the low boiler column pressure.

4. The process of any one of the preceding claims, wherein the rectifying section of the low boiler column comprises at least 8 theoretical plates.

5. The process of any one of the preceding claims, wherein the rectifying section of the low boiler column comprises internals selected from trays, random packing, or one or more structured packing elements, the structured packing elements preferably having a surface area density of at least 100 m²/m³, the structured packing elements preferably having a total height of at least 6 m.

6. The process of any one of the preceding claims, wherein the pressure at the top of the low boiler column is from 40 to 200 mbar, and the distillation temperature at the bottom of the low boiler column is from 120 to 180°C.

7. The process of any one of the preceding claims, wherein the pressure at the top of the acetate column is from 90 to 1000 mbar, and the temperature at the bottom of the acetate column is from 120 to 190°C.

8. The process of any one of the preceding claims, wherein the purified (meth)acrylic ester is withdrawn from the bottom of the low boiler column, and the purified (meth)acrylic ester is introduced into a finishing column, pure (meth)acrylic ester is withdrawn overhead from the finishing column, and high-boilers are withdrawnfrom the bottom of the finishing column.

9. The process of any one of claims 1 to 6, wherein the purified (meth)acrylic ester is withdrawn as a side stream from the low boiler column, and high-boilers are withdrawn from the bottom of the low boiler column.

10. The process of any one of the preceding claims, wherein the esterification step involves an esterification catalyst and the process comprises a catalyst removal step after the esterification step.

11. The process of any one of the preceding claims, wherein the process comprises a (meth)acrylic acid removal step prior to introducing the crude (meth)acrylic ester into the finishing column.

12. The process of any one of the preceding claims, wherein the alcohol is a C₆₋C₁₂-alkanol, preferably 2-ethylhexyl alcohol.

13. The process of any of the preceding claims, wherein acrylic acid is subjected to the esterification step.

14. The process of any of the preceding claims, wherein a C₈-alkanol, in particular a 2-ethyl-hexanol or iso-octanol, is subjected to the esterification step.

15. The process of any of the preceding claims, wherein the esterification step is carried out in the presence of an olefinic entrainer.

16. The process of claim 13, wherein the olefinic entrainer is an intrinsic by-product generated by dehydration of the alcohol.

## Patentansprüche

1. Verfahren zur Herstellung von C₆-C₁₂-Alkyl(meth)acrylsäureestern, umfassend einen Veresterungsschritt der Veresterung von (Meth)acrylsäure mit einem C₆-C₁₂-Alkohol, um einen rohen (Meth)acrylsäureester zu erhalten, wobei die (Meth)acrylsäure Spurenmengen an Essigsäure enthält; und Reinigungsschritte der Reinigung des rohen (Meth)acrylsäureesters,
wobei die Reinigungsschritte umfassen
- Einführen von rohem (Meth)acrylsäureester in die Seite einer Leichtsiederkolonne mit einem Rektifikationsabschnitt, der über der Einspeisestelle des rohen (Meth)acrylsäureesters angeordnet ist, und einem Strippabschnitt, der unter der Einspeisestelle angeordnet ist;
- Entnehmen von gereinigtem (Meth)acrylsäureester aus der Leichtsiederkolonne;
- Entnehmen einer Leichtsiederfraktion aus dem Kopf der Leichtsiederkolonne, wobei die Leichtsiederfraktion Alkohol und Essigsäureester und weniger als 10 Gew.-% (Meth)acrylsäureester umfasst;
- Leiten der Leichtsiederfraktion zu einer Acetatkolonne, wobei die Acetatkolonne bei einem Druck betrieben wird, der wenigstens 50 mbar höher als der Druck der Leichtsiederkolonne ist, und Trennen der Leichtsiederfraktion in eine an dem Kopf der Acetatkolonne entnommene Alkoholfraktion und eine an dem Sumpf der Acetatkolonne entnommene Essigsäureesterfraktion; und
- wenigstens zum Teil Rückführen der Alkoholfraktion in den Veresterungsschritt;
wobei das Verfahren keinen Rücklauf von der Acetatkolonne in die Leichtsiederkolonne umfasst.

2. Verfahren nach Anspruch 1, wobei der rohe (Meth)acrylsäureester im Wesentlichen wasserfrei ist.

3. Verfahren nach Anspruch 1 oder 2, wobei die Acetatkolonne bei einem Druck betrieben wird, der 2- bis 10-mal höher als der Druck der Leichtsiederkolonne ist.

4. Verfahren nach einem der vorstehenden Ansprüche, wobei der Rektifikationsabschnitt der Leichtsiederkolonne wenigstens 8 theoretische Böden umfasst.

5. Verfahren nach einem der vorstehenden Ansprüche, wobei der Rektifikationsabschnitt der Leichtsiederkolonne Einbauten ausgewählt aus Böden, Füllkörpern oder einem oder mehreren strukturierten Packungselementen umfasst, wobei die strukturierten Packungselemente vorzugsweise eine Oberflächendichte von wenigstens 100 m²/m³ aufweisen, wobei die strukturierten Packungselemente vorzugsweise eine Gesamthöhe von wenigstens 6 m aufweisen.

6. Verfahren nach einem der vorstehenden Ansprüche, wobei der Druck an dem Kopf der Leichtsiederkolonne von 40 bis 200 mbar beträgt und die Destillationstemperatur an dem Sumpf der Leichtsiederkolonne von 120 bis 180 °C beträgt.

7. Verfahren nach einem der vorstehenden Ansprüche, wobei der Druck an dem Kopf der Acetatkolonne von 90 bis 1000 mbar beträgt und die Temperatur an dem Sumpf der Acetatkolonne von 120 bis 190 °C beträgt.

8. Verfahren nach einem der vorstehenden Ansprüche, wobei der gereinigte (Meth)acrylsäureester aus dem Sumpf der Leichtsiederkolonne entnommen wird und der gereinigte (Meth)acrylsäureester in eine Endbehandlungskolonne eingeführt wird, reiner (Meth)acrylsäureester an dem Kopf aus der Endbehandlungskolonne entnommen wird und Hochsieder aus dem Sumpf der Endbehandlungskolonne entnommen werden.

9. Verfahren nach einem der Ansprüche 1 bis 6, wobei der gereinigte (Meth)acrylsäureester als Seitenstrom aus der Leichtsiederkolonne entnommen wird und Hochsieder aus dem Sumpf der Leichtsiederkolonne entnommen werden.

10. Verfahren nach einem der vorstehenden Ansprüche, wobei der Veresterungsschritt einen Veresterungskatalysator einbezieht und das Verfahren einen Katalysator-Entfernungsschritt nach dem Veresterungsschritt umfasst.

11. Verfahren nach einem der vorstehenden Ansprüche, wobei das Verfahren einen (Meth)acrylsäure-Entfernungsschritt vor Einführen des rohen (Meth)acrylsäureesters in die Endbehandlungskolonne umfasst.

12. Verfahren nach einem der vorstehenden Ansprüche, wobei der Alkohol ein C₆-C₁₂-Alkanol, vorzugsweise 2-Ethylhexylalkohol, ist.

13. Verfahren nach einem der vorstehenden Ansprüche, wobei Acrylsäure dem Veresterungsschritt unterworfen wird.

14. Verfahren nach einem der vorstehenden Ansprüche, wobei ein C₈-Alkanol, insbesondere ein 2-Ethylhexanol oder iso-Octanol, dem Veresterungsschritt unterworfen wird.

15. Verfahren nach einem der vorstehenden Ansprüche, wobei der Veresterungsschritt in Gegenwart eines olefinischen Schleppmittels durchgeführt wird.

16. Verfahren nach Anspruch 13, wobei das olefinische Schleppmittel ein intrinsisches Nebenprodukt, das durch Dehydratisierung des Alkohols erzeugt wird, ist.

## Revendications

1. Procédé pour la production d'esters (méth)acryliques d'alkyle en C₆-C₁₂, comprenant une étape d'estérification consistant à estérifier de l'acide (méth)acrylique avec un alcool en C₆-C₁₂ pour obtenir un ester (méth)acrylique brut, l'acide (méth)acrylique contenant des traces d'acide acétique ; et des étapes de purification consistant à purifier l'ester (méth)acrylique brut,
dans lequel les étapes de purification comprennent
- l'introduction d'ester (méth)acrylique brut dans la partie latérale d'une colonne à composés à bas point d'ébullition dotée d'une section de rectification disposée au-dessus du point d'alimentation en ester (méth)acrylique brut et d'une section de revaporisation disposée au-dessous du point d'alimentation ;
- le soutirage d'ester (méth)acrylique purifié de la colonne à composés à bas point d'ébullition ;
- le soutirage d'une fraction de composés à bas point d'ébullition de la partie supérieure de la colonne à composés à bas point d'ébullition, la fraction de composés à bas point d'ébullition comprenant de l'alcool et de l'ester acétique et moins de 10 % en poids d'ester (méth)acrylique ;
- l'envoi de la fraction de composés à bas point d'ébullition vers une colonne à acétates, la colonne à acétates étant amenée à fonctionner à une pression au moins 50 mbar plus élevée que la pression de la colonne à composés à bas point d'ébullition, et la séparation de la fraction de composés à bas point d'ébullition en une fraction d'alcool soutirée au niveau de la partie supérieure de la colonne à acétates et une fraction d'ester acétique soutirée au niveau de la partie inférieure de la colonne à acétates ; et
- le recyclage de la fraction d'alcool au moins en partie vers l'étape d'estérification ;
le procédé ne comprenant pas de recyclage de la colonne à acétates vers la colonne à composés à bas point d'ébullition.

2. Procédé selon la revendication 1, dans lequel ledit ester (méth)acrylique brut est essentiellement anhydre.

3. Procédé selon la revendication 1 ou 2, la colonne à acétates étant amenée à fonctionner à une pression 2 à 10 fois plus élevée que la pression de la colonne à composés à bas point d'ébullition.

4. Procédé selon l'une quelconque des revendications précédentes, dans lequel la section de rectification de la colonne à composés à bas point d'ébullition comprend au moins 8 plateaux théoriques.

5. Procédé selon l'une quelconque des revendications précédentes, dans lequel la section de rectification de la colonne à composés à bas point d'ébullition comprend des éléments internes choisis parmi des plateaux, un garnissage aléatoire ou un ou plusieurs éléments de garnissage structurés, les éléments de garnissage structurés ayant de préférence une surface spécifique d'au moins 100 m²/m³, les éléments de garnissage structurés ayant de préférence une hauteur totale d'au moins 6 m.

6. Procédé selon l'une quelconque des revendications précédentes, dans lequel la pression au niveau de la partie supérieure de la colonne à composés à bas point d'ébullition va de 40 à 200 mbar et la température de distillation au niveau de la partie inférieure de la colonne à composés à bas point d'ébullition va de 120 à 180 °C.

7. Procédé selon l'une quelconque des revendications précédentes, dans lequel la pression au niveau de la partie supérieure de la colonne à acétates va de 90 à 1000 mbar et la température au niveau de la partie inférieure de la colonne à acétates va de 120 à 190 °C.

8. Procédé selon l'une quelconque des revendications précédentes, dans lequel l'ester (méth)acrylique purifié est soutiré de la partie inférieure de la colonne à composés à bas point d'ébullition et l'ester (méth)acrylique purifié est introduit dans une colonne de finissage, de l'ester (méth)acrylique pur est soutiré en tête de la colonne de finissage et des composés à haut point d'ébullition sont soutirés de la partie inférieure de la colonne de finissage.

9. Procédé selon l'une quelconque des revendications 1 à 6, dans lequel l'ester (méth)acrylique purifié est soutiré sous forme d'un flux latéral de la colonne à composés à bas point d'ébullition et des composés à haut point d'ébullition sont soutirés de la partie inférieure de la colonne à composés à bas point d'ébullition.

10. Procédé selon l'une quelconque des revendications précédentes, dans lequel l'étape d'estérification met en jeu un catalyseur d'estérification et le procédé comprend une étape d'élimination de catalyseur après l'étape d'estérification.

11. Procédé selon l'une quelconque des revendications précédentes, le procédé comprenant une étape d'élimination d'acide (méth)acrylique avant l'introduction de l'ester (méth)acrylique brut dans la colonne de finissage.

12. Procédé selon l'une quelconque des revendications précédentes, dans lequel l'alcool est un alcanol en C₆-C₁₂, de préférence l'alcool 2-éthylhexylique.

13. Procédé selon l'une quelconque des revendications précédentes, dans lequel de l'acide acrylique est soumis à l'étape d'estérification.

14. Procédé selon l'une quelconque des revendications précédentes, dans lequel un alcanol en C₈, en particulier du 2-éthylhexanol ou de l'isooctanol, est soumis à l'étape d'estérification.

15. Procédé selon l'une quelconque des revendications précédentes, dans lequel l'étape d'estérification est effectuée en présence d'un entraîneur oléfinique.

16. Procédé selon la revendication 13, dans lequel l'entraîneur oléfinique est un sous-produit intrinsèque généré par déshydratation de l'alcool.
